# EUROPÄISCHE PATENTSCHRIFT

(11) **EP 1 829 508 B1**
(45) Veröffentlichungstag und Bekanntmachung des Hinweises auf die Patenterteilung: **10.12.2008**
(21) Anmeldenummer: 06026136.9
(22) Anmeldetag: 18.12.2006
(51) Int. Cl.: A61F 5/37

(54) **Orthopädisches Gilet**
Orthopedic vest
Gilet orthopedique

(30) Priorität: 01.03.2006 CH 3192006
(43) Veröffentlichungstag der Anmeldung: 05.09.2007
(73) Patentinhaber: HOMED AG, 4712 Laupersdorf (CH)
(72) Erfinder: Hodel, Marianne, 4712 Laupersdorf (CH)
(74) Vertreter: Schöneborn, Holger

(56) Entgegenhaltungen:
- EP-A1- 0 694 296
- WO-A-82/03767
- CH-A5- 689 108
- CH-A5- 694 612

## Beschreibung

Es wird ein orthopädisches Gilet beschrieben, das eingesetzt wird bei extrem starken

Verletzungen im Schulter- und Oberarmbereich. Die vorliegende Erfindung geht auf ein orthopädisches Giletzurück, wie es in der CH-689 108 beschrieben wird. Das Gilet ist in Anspruch 1 beschrieben und besteht aus einem um den Oberkörper gelegten breiten Gürtel, der die ganze Brust und das Schultergelenk abdeckt und im Schulterbereich eine Öffnung (1) aufweist, welche je nach Variante mit Reissverschluss oder Hakenband geöffnet oder geschlossen werden kann. Dies hat zum Vorteil, dass die Wunde im Schulterbereich behandelt werden kann, ohne dass das Gilet ausgezogen werden muss.

Sämtliche Verschlussteile des Gilets sind auf der Vorder- und Rückseite mit einem Hakenband versehen (2). Das Gilet kann somit links oder rechts getragen werden, indem man das nicht benötigte Verschlussteil (2) auf das aktive Teil umlegt.
Das Rückenteil (3) ist zusätzlich mit einem Stoffgürtel (4) mit Verschlussteilen (2) versehen, damit dieser zur Verbindung zum Vorderteil (5) festgezogen werden kann und damit eine rutschfeste Haltung in der Taille gewährleistet. Im Brustbereich des Vorderteils (5) ist zusätzlich ein 10 cm breiter, erweiterter Brusteinsatz (6) eingenäht, das bei weiblichen Personen mehr Platz für die Brust offen lässt, bei männlichen Personen den Tragekomfort jedoch nicht beeinträchtigt. Der untere Teil des Vorderteils (5) besteht aus einer verlängerten Armtrageschlinge (7), die bei geschlossenem Zustand dafür sorgt, dass die Hand nicht frei herunterhängt, sondern eine bequeme Abstützung des Handballens gewährleistet.
An der Verbindung zwischen Vorderteil (5) und Rückenteil (3) wird ein Ellbogenteil (8) angenäht, der mit Umschlagen auf den Oberarm den Ellbogen frei lässt oder mit Umschlagen auf den Unterarm über den Ellbogen diesen abdeckt.

Das Trägermaterial besteht aus 3 mm dickem Polyurethan-Schaumstoff, der beidseitig mit Polyamid-Velours-Stoff flammkaschiert wird, d.h. unter Wärmeeinwirkung untrennbar zusammengepresst ist. Zusätzlich ist das gesamte Trägermaterial mit einem Abstand von 15 mm diametral mit 3 mm grossen durchgestanzten Löchern versehen, damit die Körperpartien unter dem Gilet besser atmen können.

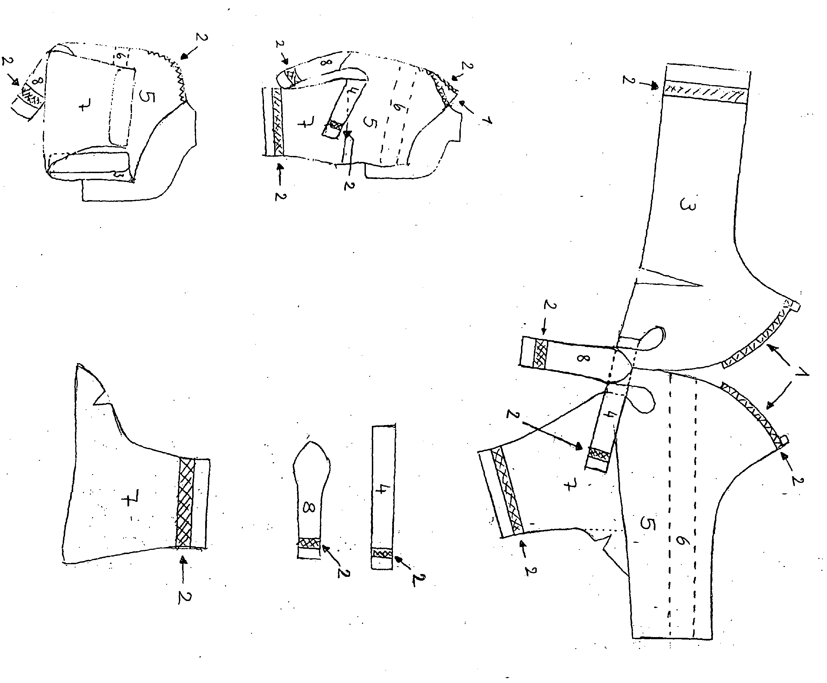

## Patentansprüche

1. Orthopädisches Gilet zur Ruhigstellung und Fixierung der Schulter und des Oberarmes bei extremen Verletzungen, welches als einen Vorderteil und einen Rückenteil aufweisenden Gürtel gefertigt ist, der zum Verschließen an beiden Enden mit einem Hakenband (2) versehen ist und im Übergangsbereich zwischen Vorderteil (5) und Rückenteil (3) einen Schulterteil aufweist, **dadurch gekennzeichnet, dass** der Schulterteil zum Freigeben der Schulter eine Öffnung (1) aufweist, welche je nach Variante mit eingenähtem Reißverschluss oder Hakenband geöffnet oder geschlossen werden kann, und der Rückenteil (3) zusätzlich mit einem Stoffgürtel (4) mit Verschlussteilen (2) versehen ist, der zur Herstellung einer Verbindung zum Vorderteil (5) und zur Gewährleistung einer rutschfesten Halterung in der Taille geeignet ist.

2. Orthopädisches Gilet nach Anspruch 1, **dadurch gekennzeichnet, dass** der Reißverschluss oder Hakenband in der Öffnung (1) so verdeckt eingenäht ist, dass er auch bei Fixierung der linken oder rechten Schulter unsichtbar ist.

3. Orthopädisches Gilet nach Anspruch 1, **dadurch gekennzeichnet, dass** im Brustbereich des Vorderteils (5) ein 15 cm breiter Brusteinsatz (6) eingenäht ist, das bei weiblichen Personen mehr Platz für die Brust offen lässt, bei männlichen Personen den Tragekomfort jedoch nicht beeinträchtigt.

4. Orthopädisches Gilet nach einem der vorhergehenden Ansprüche, **dadurch gekennzeichnet, dass** am Vorderteil (5) eine verlängerte Armtragschlinge (7) besteht, die dafür sorgt, dass die Hand des verletzten Körperteils (Schulter oder Oberarm) nicht frei herunterhängt, sondern eine bequeme Abstützung des Handballens gewährleistet.

## Claims

1. An orthopaedic waistcoat for immobilising and fixing the shoulder and the upper arm in the case of extreme injuries, which is made in the form of a belt which has a front portion and a rear portion and which is provided with a hook-and-loop tape for closure at both ends and which has a shoulder portion in the transitional region between the front portion (5) and the rear portion (3), **characterised in that** the shoulder portion for exposing the shoulder has an opening (1) which depending on the respective variant can be opened or closed with a sewn-in zip fastener or hook-and-loop tape, and the rear portion (3) is additionally provided with a material belt (4) having closure portions (2), which is suitable for making a connection to the front portion (5) and for ensuring a non-slip hold at the waist.

2. An orthopaedic waistcoat according to claim 1 **characterised in that** the zip fastener or hook-and-loop tape is sewn in the opening (1) in concealed fashion in such a way that it is invisible even when fixing the left or right shoulder.

3. An orthopaedic waistcoat according to claim 1 **characterised in that** sewn in the chest region of the front portion (5) is a 15 cm wide chest insert (6) which in the case of females leaves more space open for the breast but which in the case of males does not adversely affect wearing comfort.

4. An orthopaedic waistcoat according to one of the preceding claims **characterised in that** on the front portion (5) there is an extended arm support sling (7) which ensures that the hand of the injured part of the body (shoulder or upper arm) does not hang down freely but ensures comfortable support for the ball of the thumb.

## Revendications

1. Gilet orthopédique pour l'immobilisation et la fixation de l'épaule et de la partie supérieure du bras lors de blessures graves, qui est réalisé sous la forme d'une ceinture présentant une partie antérieure et une partie postérieure, qui est munie à ses deux extrémités d'une bande agrippante (2) pour la fermeture et présente une partie pour l'épaule dans la zone intermédiaire entre la partie antérieure (5) et la partie postérieure (3), **caractérisé en ce que** la partie pour l'épaule présente pour laisser l'épaule libre une ouverture (1), qui selon la variante peut être ouverte ou fermée avec une fermeture à glissière ou une bande agrippante cousues, et la partie postérieure (3) est en outre munie d'une ceinture en tissu (4) ayant des éléments de fermeture (2), qui convient pour la réalisation d'une liaison à la partie antérieure (5) et pour garantir un maintien à la taille sans glissement.

2. Gilet orthopédique selon la revendication 1, **caractérisé en ce que** la fermeture à glissière ou la bande agrippante dans l'ouverture (1) est cousue avec un recouvrement tel qu'elle est invisible lors de la fixation de l'épaule aussi bien gauche que droite.

3. Gilet orthopédique selon la revendication 1, **caractérisé en ce que** dans la zone de poitrine de la partie antérieure (5) est cousu un plastron (6) ayant une largeur de 15 cm, qui libère plus de place pour la poitrine chez les personnes de sexe féminin, mais ne gêne pas le confort du porteur pour les personnes de sexe masculin.

4. Gilet orthopédique selon l'une des revendications précédentes, **caractérisé en ce qu'**il se trouve sur la partie antérieure (5) une écharpe allongée pour supporter le bras (7), qui veille à ce que la main de la partie blessée du corps (épaule ou avant-bras) ne pende pas librement, mais assure au contraire un soutien confortable des éminences thénar et hypothénar de la main.
